(19)

Europäisches
Patentamt
European
Patent Office
Office européen
des brevets

(11)     **EP 2 841 942 B1**

(12)                                **EUROPEAN PATENT SPECIFICATION**

(45) Date of publication and mention
of the grant of the patent:
**02.01.2019   Bulletin 2019/01**

(51) Int Cl.:
**G01N 33/50** [(2006.01)]

(21) Application number: **13781717.7**

(22) Date of filing: **24.04.2013**

(86) International application number:
**PCT/US2013/038025**

(87) International publication number:
**WO 2013/163318 (31.10.2013 Gazette 2013/44)**

(54) **PRE-ECLAMPSIA SCREENING METHODS**

PRÄEKLAMPSIE-SCREENING-VERFAHREN

MÉTHODES DE DÉPISTAGE D'UNE PRÉ-ÉCLAMPSIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.04.2012   US 201261637637 P**

(43) Date of publication of application:
**04.03.2015   Bulletin 2015/10**

(73) Proprietor: **Siemens Healthcare Diagnostics, Inc.
Tarrytown, NY 10591 (US)**

(72) Inventors:
• **GRATACOS, Eduard
E-08022 Barcelona (ES)**
• **FIGUERAS, Francesc
E-08348 Cabrils (ES)**
• **CRISPI, Fatima
E-08025 Barcelona (ES)**
• **LAS ALVIA, Luis
White Plains, NY 10601 (US)**
• **MEENAN, James
West Grove, PA 19390 (US)**

(74) Representative: **Schweitzer, Klaus et al
Plate Schweitzer Zounek
Patentanwälte
Rheingaustrasse 196
65203 Wiesbaden (DE)**

(56) References cited:
**WO-A1-2010/059952     WO-A2-00/78330
US-A1- 2010 304 412**

• **ASMA KHALIL ET AL: "First trimester markers for
the prediction of pre-eclampsia in women with a
priori high risk", ULTRASOUND IN OBSTETRICS
AND GYNECOLOGY, 1 January 2010
(2010-01-01), pages n/a-n/a, XP055237632, GB
ISSN: 0960-7692, DOI: 10.1002/uog.7559**
• **L. C. Y. POON ET AL: "Hypertensive disorders in
pregnancy: screening by uterine artery Doppler
at 11-13 weeks", ULTRASOUND IN OBSTETRICS
AND GYNECOLOGY, vol. 34, no. 2, 1 January 2009
(2009-01-01), pages 142-148, XP055173939, ISSN:
0960-7692, DOI: 10.1002/uog.6452**
• **DUCKITT ET AL.: 'Risk factors for pre-edampsia
at antenatal booking: systematic review of
controlled studies' BRITISH MEDICAL JOURNAL
02 March 2005, pages 1 - 7, XP055173937**
• **POON ET AL.: 'Hypertensive disorders in
pregnancy: combined screening by uterine artery
Doppler, blood pressure and serum PAPP-A at
11-13 weeks' PRENATAL DIAGNOSIS vol. 34, no.
2, 27 January 2010, pages 142 - 148, XP055173939**
• **AKOLEKAR ET AL.: 'Prediction of early,
intermediate and late pre-eclampsia from
maternal factors, biophysical and biochemical
markers at 11-13 weeks' PRENATAL DIAGNOSIS
vol. 31, 01 January 2011, pages 66 - 74,
XP055161425**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

**FIELD OF INVENTION**

The present invention relates generally to diagnostic methods for treating early onset pre-eclampsia, as well as to methods of screening for and predicting the likelihood that a pregnant female patient will develop early pre-eclampsia, by assessing specific combinations of factors.

**BACKGROUND OF THE INVENTION**

Pre-eclampsia (PE) occurs in approximately 2-3% of pregnancies. In developed countries, PE is the primary cause of maternal admission to intensive care units and causes approximately 15% of all pregnancy-related deaths. Additionally, PE is associated with an increased risk of perinatal mortality and is the cause for approximately 10% of stillbirths and 15% of preterm births.

PE is generally characterized by hypertension and proteinuria after 20 weeks of gestation in a previously normotensive woman, although this definition varies among different organizations. The disease has been further subdivided into mild, moderate, and severe PE, as well early and late onset PE. Early onset PE is is the clinical form that contributes most significantly to adverse outcomes. The precise etiopathology remains the subject of intensive research, though it is believed to be multifactorial. Research suggests that the presence of the placenta, rather than the fetus, is responsible for the development of pre-eclampsia.

Recent studies suggest that various factors may have predictive value in assessing the likelihood that an individual will develop PE, including maternal history, biochemical markers, and biophysical markers in the first trimester of pregnancy, a period in which prophylactic strategies may be most effective. The use of various factors to aid in predicting early and late onset PE have had varying rates of success.

The variability in detection rates from previous studies is likely due in part to methodological limitations, and in particular, because of the specific, limited populations of pregnant women that have been assessed in these studies. For example, many studies investigating the predictive value of certain factors have used a study population located in the UK. The selection of this study population has likely had a large impact on the outcome and applicability of the data, because, for example, in southern European countries, the rates of obesity and chronic hypertension are lower than in the UK. Furthermore, many previous studies have made assessments late in the first trimester, which can also influence the extent to which certain measured factors can predict the onset of the condition.

There is a need therefore, for effective first trimester screening methods for both early and late onset PE, so that appropriate management of PE patients can be initiated as soon as possible.

**SUMMARY OF THE INVENTION**

In accordance with the foregoing objectives and others, the present invention identifies unique combinations of factors that can predict the likelihood of a pregnant female patient developing early or late onset pre-eclampsia. The assessment of these factors can be used clinically in screening tests for early and late onset pre-eclampsia, which can potentially alter the course of treatment for such patients.

In one aspect of the invention, diagnostic methods of screening for pregnant female patients with a likelihood of developing early pre-eclampsia are provided, comprising the steps of determination of *a priori* risk, *a posteriori* risk, and determining whether the *a posteriori* risk exceeds a pre-determined threshold. The methods are provided for determining the need for treating a pregnant female patient comprising determining the *a priori* risk of developing early onset pre-eclampsia (early pre-clampsia), and determining the *a posteriori* risk of developing early pre-eclampsia.

Determining the *a priori risk* of developing early pre-eclampsia in a pregnant female patient may include the assessment of various maternal factors, such as previous pre-eclampsia, chronic hypertension, and renal disease. Determining the *a posteriori risk* of developing early pre-eclampsia in a pregnant female patient may include the assessment of various patient-specific factors, such as mean uterine artery pulsatility index (UtA-PI), mean arterial pressure (MAP), and *a priori* risk of developing early pre-eclampsia.

In the methods of the invention, the *a priori* risk of developing early pre-eclampsia may be calculated utilizing coefficients for each of the maternal factors (binary variables), the coefficients being generated utilizing logistic regression analysis. The *a posteriori* risk of developing early pre-eclampsia may be calculated utilizing coefficients for each of the patient-specific factors, the coefficients being generated utilizing logistic regression analysis.

In one embodiment, the *a posteriori* risk of developing early pre-eclampsia is determined by $e^Y/[1 + e^Y]$, where Y is calculated as -0.363 + (2.653 log *a priori* risk) + (12.88 x log multiple of the expected normal median (MoM) mean UtA-PI) + (25.915 x log MoM mean arterial pressure); and where said *a priori* risk is determined by $e^{Y'}/[1 + e^{Y'}]$, where Y' is calculated as -5.617 + (2.659 if previous pre-eclampsia) + (1.729 if chronic hypertension) + (3.765 if renal disease).

The methods of the invention also comprise determining whether the *a posteriori* risk of developing early pre-eclampsia exceeds a pre-determined threshold risk.

A treatment may be provided to the pregnant female patient if the calculated *a posteriori* risk exceeds the pre-determined threshold risk. In some embodiments the detection rate for detecting early pre-eclampsia by the methods of the invention is at least about 80%, with a false positive rate of about 10%. In other embodiments, the detection rate for detecting early pre-eclampsia by the methods of the invention is at least about 90%, with a false positive rate of about 15%.

The treatment provided to a patient determined to have an *a posteriori* risk above the pre-determined threshold risk may include one or more of the following: administering a pharmaceutical agent to the patient to prevent, treat, reduce the severity of, or delay the onset of pre-eclampsia or eclampsia; implementing a change in diet or nutrition; inducing labor; performing a Cesarean-section; increasing the frequency of blood pressure measurements for said patient; increasing the frequency of blood testing; and increasing the frequency of fetal monitoring.

In one aspect of the invention, the patient-specific factors that are assessed do not comprise one or more of placental growth factor (PIGF), vascular endothelial growth factor (VEGF), free β-human chorionic gonadotropin (fβ-hCG), placental protein 13 (PP13), Inhibin A, a disintegrin and metalloprotease-12 (ADAM12), or anti-angiogenic proteins including soluble endoglin (sEng) and the soluble truncated form of the full-length VEGF receptor type-1 (sFltl).

In another aspect of the invention, the patient-specific factors that are assessed do not comprise biochemical markers.

In yet another aspect (not according to the invention), methods are provided for treating a pregnant female patient comprising determining the *a priori* risk of developing late onset pre-eclampsia (late pre-clampsia), and determining the *a posteriori* risk of developing late pre-eclampsia.

Determining the *a priori risk* of developing late pre-eclampsia in a pregnant female patient may include the assessment of various maternal factors, such as previous pre-eclampsia, chronic hypertension, diabetes mellitus, thrombophilic condition, multiparity, and body mass index (BMI). Determining the *a posteriori risk* of developing late pre-eclampsia in a pregnant female patient may include the assessment of various patient-specific factors, such as pregnancy-associated plasma protein-A (PAPP-A) and the *a priori* risk of developing late pre-eclampsia.

In some embodiments, the determination of the maternal factors, the patient-specific factors, or both, further comprise obtaining a biological sample from the patient. The biological sample may be obtained in order to determine levels of, or the presence of, one or more biomarkers. In one embodiment (not according to the invention), the biomarkers comprise PAPP-A.

The methods of the invention also comprise determining whether the *a posteriori* risk of developing late pre-eclampsia exceeds a pre-determined threshold risk, the pre-determined threshold being generated by statistical analyses such as logistic regression.

In the methods (not according to the invention), the *a priori* risk of developing late pre-eclampsia may be calculated utilizing coefficients for each of the maternal factors (binary variables), the coefficients being generated utilizing logistic regression analysis. The *a posteriori* risk of developing late pre-eclampsia may be calculated utilizing coefficients for each of the patient-specific factors, the coefficients being generated utilizing logistic regression analysis.

In one embodiment (not according to the invention), the *a posteriori* risk of developing late pre-eclampsia is determined by $e^Y/[1 + e^Y]$, where Y is calculated as 0.328 + (2.205 x log *a priori* risk) - (1.307 x log MoM PAPP-A); and where said *a priori* risk is determined by $e^{Y'}/[1 + e^{Y'}]$, where Y' is calculated as -6.135 + (2.124 if previous pre-eclampsia) + (1.571 if chronic hypertension) + (0.958 if diabetes mellitus) + (1.416 if thrombophilic condition) - (0.487 if multiparae) + (0.093 x BMI).

The methods of the invention also comprise determining whether the *a posteriori* risk of developing late pre-eclampsia exceeds a pre-determined threshold risk.

A treatment may be provided to the pregnant female patient if the calculated *a posteriori* risk of developing late pre-eclampsia exceeds the pre-determined threshold risk. The treatment provided to a patient determined to have *a posteriori* risk above the pre-determined threshold risk may include one or more of the following: administering a pharmaceutical agent to the patient to prevent, treat, reduce the severity of, or delay the onset of pre-eclampsia or eclampsia; implementing a change in diet or nutrition; inducing labor; performing a Cesarean-section; increasing the frequency of blood pressure measurements for said patient; increasing the frequency of blood testing; and increasing the frequency of fetal monitoring.

In one aspect of the invention, the patient-specific factors that are assessed do not comprise one or more of placental growth factor (PIGF), vascular endothelial growth factor (VEGF), free β-human chorionic gonadotropin (fβ-hCG), placental protein 13 (PP13), Inhibin A, a disintegrin and metalloprotease-12 (ADAM12), or anti-angiogenic proteins including soluble endoglin (sEng) and the soluble truncated form of the full-length VEGF receptor type-1 (sFltl).

In other embodiments, the invention provides methods of screening for pregnant female patients with a likelihood of developing early or late pre-eclampsia comprising the steps described herein to determine *a priori* risk, *a posteriori* risk, and determining whether the *a posteriori* risk exceeds a pre-determined threshold risk. In yet other embodiments, the invention provides methods of predicting the likelihood that a pregnant female will develop early or late pre-eclampsia, comprising the steps described herein to determine *a priori* risk, *a posteriori* risk, and determining whether the *a posteriori* risk exceeds a pre-determined threshold risk.

These and other aspects of the invention will become apparent to those skilled in the art after a reading of the following detailed description of the invention, including the figures and appended claims.

## BRIEF DESCRIPTION OF THE FIGURES

FIG. 1A. Box-plot of log MoM of mean uterine artery Doppler pulsatility indices (UtA-PI) among the study groups. PE: preeclampsia. MoM: multiple of the expected normal median.

FIG. 1B. Box-plot of log MoM of mean arterial pressure (MAP) among the study groups. PE: preeclampsia. MoM: multiple of the expected normal median.

FIG. 1C. Box-plot of log MoM of pregnancy associated plasma protein-A (PAPP-A) levels among the study groups. PE: preeclampsia. MoM: multiple of the expected normal median.

FIG. 2. Receiver operator characteristic curves for late (thin line) and early PE (thick line).

## DETAILED DESCRIPTION

In its broadest sense, the present invention relates to diagnostic methods for treating pregnant female patients at risk of developing early pre-eclampsia, based on the results of screening tests that assess specific maternal factors relating to medical and obstetric history, as well as factors that are obtained from the patient in the first trimester of pregnancy. The invention also relates to diagnostic methods for screening for risk of early and late pre-eclampsia, as well as to methods of predicting the likelihood of developing early and late pre-eclampsia.

The methods of the invention provide determining the need for providing treatments for pregnant female patients in need of treatment based on a calculated risk of developing early and/or late pre-eclampsia. Methods for determining both the *a priori* risk of developing early and late pre-eclampsia and the *a posteriori* risk of developing early and late pre-eclampsia are encompassed by the invention. The need for providing a treatment to patients is determined if the a posteriori risk of developing early and/or late pre-eclampsia exceeds a pre-determined threshold risk.

The methods of the invention are contemplated to cover all forms of pre-eclampsia, and all signs and symptoms thereof. Pre-eclampsia refers to a syndrome characterized by the onset of hypertension and proteinuria after 20 weeks of gestation in a previously normotensive woman. Additional symptoms of pre-eclampsia can include headaches, visual disturbances, epigastric pain, thrombocytopenia, and abnormal liver function. Pre-eclampsia can be considered severe when severe hypertension, severe proteinuria, or other signs and symptoms of end-organ injury are present. In the absence of such findings, pre-eclampsia is often referred to as mild, although moderate pre-eclampsia can refer to pre-eclampsia with symptoms the severity of which are in between those of the severe and the mild form of the disease.

As used herein, early pre-eclampsia refers to pre-eclampsia developing before 34 weeks of gestation, and late pre-eclampsia refers to pre-eclampsia developing at 34 weeks of gestation or later. Eclampsia refers to the development of grand mal seizures in a woman with eclampsia in the absence of other neurological conditions that could account for such seizures.

In some embodiments, pre-eclampsia is diagnosed as systolic blood pressure (BP) of greater than or equal to about 140 mmHg and/or diastolic BP of greater than or equal to about 90 mmHg on at least two occasions, four hours apart, developing after 20 weeks of gestation in a previously normotensive woman, as well as proteinuria of greater than about 300 mg in a 24-hour urine specimen. In some embodiments, however, the patient may have a previous history of hypertension, which may be referred to as chronic or pre-existing hypertension.

The *a priori* risk of developing early or late pre-eclampsia describes the risk of a pregnant woman developing early or late pre-eclampsia based on maternal factors that can be obtained during the first trimester of pregnancy, or that can be obtained prior to pregnancy. In one embodiment, the maternal factors are obtained at about 8 weeks to about 10 weeks of pregnancy.

For assessing the *a priori* risk of developing early pre-eclampsia, the maternal factors may comprise, in one embodiment, previous pre-eclampsia, chronic hypertension, and renal disease. In another embodiment, the maternal factors may consist of previous pre-eclampsia, chronic hypertension, and renal disease.

For assessing the *a priori* risk of developing late pre-eclampsia, the maternal factors may comprise, in one embodiment (not according to the invention), pre-eclampsia, chronic hypertension, diabetes mellitus, thrombophilic condition, multiparity, and body mass index (BMI). In another embodiment (not according to the invention), the maternal factors may consist of pre-eclampsia, chronic hypertension, diabetes mellitus, thrombophilic condition, multiparity, and body mass index (BMI).

The *a posteriori* risk of developing early or late pre-eclampsia describes the risk of a pregnant woman developing early or late pre-eclampsia based on patient-specific factors that may be obtained from a pregnant woman during the first trimester of pregnancy. In one embodiment, the patient-specific factors are obtained at about 8 weeks to about 10 weeks of pregnancy.

For assessing the *a posteriori* risk of developing early pre-eclampsia, the patient-specific factors may comprise, in one embodiment, mean uterine artery pulsatility index (UtA-PI), mean arterial pressure (MAP), and the *a priori* risk of developing early pre-eclampsia. In another embodiment, the patient-specific factors consist of mean uterine artery pulsatility index (UtA-PI), mean arterial pressure (MAP), and the *a priori* risk of developing early pre-eclampsia.

In one embodiment, the patient specific factors utilized in determining the *a posteriori* risk of developing early pre-eclampsia do not comprise placental growth factor (PIGF), vascular endothelial growth factor (VEGF), free β-human chorionic gonadotropin (fβ-hCG), placental protein 13 (PP13), Inhibin A, a disintegrin and metalloprotease-12 (ADAM 12), or anti-angiogenic proteins including soluble endoglin (sEng) and the soluble truncated form of the full-length VEGF receptor type-1 (sFltl).

In some embodiments, the patient specific factors utilized in determining the *a posteriori* risk of developing early pre-eclampsia do not comprise biochemical markers.

For assessing the *a posteriori* risk of developing late pre-eclampsia, the patient-specific factors may comprise pregnancy-associated plasma protein-A (PAPP-A) and the *a priori* risk of developing late pre-eclampsia. The patient-specific factors may consist of pregnancy-associated plasma protein-A (PAPP-A) and the *a priori* risk of developing late pre-eclampsia.

In one embodiment, the patient specific factors utilized in determining the *a posteriori* risk of developing late pre-eclampsia do not comprise placental growth factor (PIGF), vascular endothelial growth factor (VEGF), free β-human chorionic gonadotropin (fβ-hCG), placental protein 13 (PP13), Inhibin A, a disintegrin and metalloprotease-12 (ADAM 12), or anti-angiogenic proteins including soluble endoglin (sEng) and the soluble truncated form of the full-length VEGF receptor type-1 (sFltl).

In some embodiments, determining the *a posteriori* risk of developing late pre-eclampsia further comprises obtaining a biological sample from the patient. Obtaining a biological sample from the patient may comprise, for example, drawing blood from the patient, collecting a urine sample from the patient, or collecting other biological material or tissue, for biochemical measurements. The biological sample may be obtained in order to determine levels of, or the presence of, one or more biomarkers. In one embodiment (not according to the invention), the biomarkers comprise PAPP-A.

When the *a posteriori* risk of developing early and/or late pre-eclampsia exceeds a pre-determined threshold risk, the patient may receive one or more treatments to prevent, treat, reduce the severity of, or delay the onset of early and/or late onset pre-eclampsia. The choice of treatment or combination of treatments suitable for a given patient will be apparent to one skilled in the art, the decision potentially taking into account the assessment of one or more clinical factors from the mother and/or the fetus. The treatment may be selected from administering a pharmaceutical agent to said patient to prevent, treat, reduce the severity of, or delay the onset of pre-eclampsia or eclampsia; implementing a change in diet or nutrition; inducing labor; performing a Cesarean-section; increasing the frequency of blood pressure measurements for said patient; increasing the frequency of blood testing; and increasing the frequency of fetal monitoring.

Pharmaceutical agents administered to the patient to prevent, treat, reduce the severity of, or delay the onset of pre-eclampsia or eclampsia may comprise one or more of antihypertensive agents such as Labetolol or Nifedipine; anti-seizure medication agents such as magnesium sulfate; antenatal corticosteroids; and aspirin.

The treatment may comprise hospitalization of the patient.

The treatment administered to the patient may comprise a specific monitoring protocol for monitoring the patient and/or the fetus, and/or an increase in monitoring various parameters and factors from the patient and/or the fetus. In some embodiments, maternal blood pressure monitoring is performed at least every four hours. In other embodiments, maternal blood pressure monitoring is performed at least every one to two hours. In some embodiments, maternal monitoring comprises frequent laboratory studies, non-limiting examples including complete blood count (including platelet count and smear); electrolytes; creatinine; alanine and aspartate aminotransferase; lactic acid dehydrogenase; albumin; coagulatory profile (prothrombin time (PT), partial thromboplastin time (PPT), fibrinogen); and liver function tests. Laboratory studies may be performed once daily, twice daily, there times daily, four times daily, five times daily, once every other day, or once weekly, for example.

In some embodiments, maternal monitoring may comprise frequent assessment of maternal symptoms, including once daily, twice daily, three times daily, four times daily, five times daily, or six times daily. In some embodiments, assessment of maternal symptoms may take place once per week, twice per week, three times per week, four times per week, or five times per week. Maternal symptoms may comprise headache, vision changes, epigastric or abdominal pain, decreased fetal activity, vaginal bleeding, pulmonary edema, renal failure, severe thrombocytopenia, HELLP syndrome (Hemolysis, Elevated Liver enzymes, and Low Platelet count), preterm labor; preterm premature rupture of membranes; and altered mental state. Maternal monitoring may also comprise frequent assessment of fluid intake and urine output.

In some embodiments, fetal monitoring may comprise frequent assessment of fetal well-being, comprising assessing fetal kick count, nonstress tests, amniotic fluid volume determination, estimation of fetal growth, umbilical artery Doppler velocimetry, and fetal heart rate assessment. Assessments of the fetus may take place once per week, twice per week, three times per week, four times per week, or five times per week. In some embodiments, fetal assessment may take place once daily, twice daily, three times daily, four times daily, five times daily, or six times daily.

Treatments may also comprise implementing a change in diet or nutrition, which may comprise, for example, calcium supplementation, selenium supplementation, and antioxidant supplementation (such as Vitamins A and C). Such treatment may also include nutritional replacement, in cases of proteinuria, thereby replacing proteins excreted in the urine.

The treatment may comprise obtaining a biological sample from the patient or from the fetus, such as, for example, drawing a blood sample or collecting a urine sample for biochemical measurements, obtaining a urine sample to measure urine output, or obtaining an amniotic fluid sample.

The treatment may comprise delivery of the fetus, which can be performed by any methods known to those skilled in the art, such as induction of labor via vaginal delivery or Cesarean section.

The methods of the invention comprise determining the *a posteriori* risk of developing early or late pre-eclampsia, and comparing that risk to a pre-determined threshold risk. In one embodiment, the methods of the invention further comprise a step of printing the results, or displaying the results on a computer, and providing the printed or displayed results to the patient, to a physician, or to a caregiver. The results printed or displayed comprise one or more the *a priori* risk of developing early and/or late pre-eclampsia, the *a posteriori* risk of developing early and/or late pre-eclampsia, a comparison of the *a posteriori risk* and the pre-determined threshold risk, and whether or not the *a posteriori* risk exceeds the pre-determined threshold risk.

A treatment may be administered to a patient if the *a posteriori* risk for early and/or late pre-eclampsia exceeds a pre-determined threshold risk. In some embodiments, the pre-determined threshold risk is about 1 in 5, about 1 in 15, about 1 in 25, about 1 in 35, about 1 in 45, about 1 in 55, about 1 in 65, about 1 in 75, about in 85, about 1 in 100, about 1 in 125, about 1 in 150, about 1 in 175, or about 1 in 200.

The methods of the invention comprise determining the *a priori* risk and the *a posteriori* risk of a patient developing early pre-eclampsia. The determination takes into account the specific maternal and patient-specific factors described herein, utilizing suitable statistical methodology known to those skilled in the art. In one embodiment, the *a priori* risk is calculated utilizing coefficients for each of the maternal factors, the coefficients being generated utilizing, for example, logistic regression analysis. In another embodiment, the *a posteriori* risk is calculated utilizing coefficients for each of the patient-specific factors, the coefficients being generated utilizing, for example, logistic regression analysis.

In deriving specific models to determine the *a priori* and *a posteriori* risk utilizing appropriate statistical tests known to those skilled in the art, in some embodiments (not according to the invention), levels of biochemical markers, such as PAPP-A may be converted to multiples of the expected normal median (MoM), which then may be corrected for factors comprising, for example, crown-rump length, maternal age, body-mass index, smoking and diabetes status, and ethnicity.

Logarithmic transformation may be performed to normalize values such as uterine artery pulsatility index and mean arterial pressure.

Receiver operator characteristic (ROC) curves may be generated to analyze the performance of the models.

In some embodiments of the invention, the detection rate of determining whether or not a patient will develop early pre-eclampsia based on the methods of the invention is at least about 90% with a false positive rate of about 15%, in another rembodiment the detection rate is at least about 80% with a false positive rate of about 10%, and in yet another embodiment, the detection rate is at least about 70% with a false positive rate of about 5%.

In some embodiments of the invention, the detection rate of determining whether or not a patient will develop late pre-eclampsia based on the methods of the invention is at least about 40% with a false positive rate of about 15%, in another embodiment the detection rate is at least about 40% with a false positive rate of about 10%, and in yet another embodiment, the detection rate is at least about 30% with a false positive rate of about 5%.

In one embodiment, the *a posteriori* risk of developing early pre-eclampsia is determined by calculating: $e^Y/[1 + e^Y]$, where Y is calculated as -0.363 + (2.653 log *a priori* risk) + (12.88 x log MoM mean UtA-PI) + (25.915 x log MoM mean arterial pressure); and where said *a priori* risk is determined by calculating: $e^{Y'}/[1 + e^{Y'}]$, where Y' is calculated as - 5.617 + (2.659 if previous pre-eclampsia) + (1.729 if chronic hypertension) + (3.765 if renal disease). In determining the *a priori* risk in the formula, a value of 2.659 is added to Y' if the patient has had pre-eclampsia previously, and a value of 0 is added to Y' if the patient has not had pre-eclampsia previously. In addition, a value of 1.729 is added to Y' if the patient has a history of chronic hypertension, and a value of 0 is added to Y' if the patient does not have a history of chronic hypertension. Further, a value of 3.765 is added to Y' if the patient has rental disease, and a value of 0 is added to Y' if the patient does not have renal disease.

In one embodiment (not according to the invention), the *a posteriori* risk of developing late pre-eclampsia is determined by calculating: $e^Y/[1 + e^Y]$, where Y is calculated as 0.328 + (2.205 x log *a priori* risk) - (1.307 x log MoM PAPP-A); and where said *a priori* risk is determined by calculating: $e^{Y'}/[1 + e^{Y'}]$, where Y' is calculated as -6.135 + (2.124 if previous pre-eclampsia) + (1.571 if chronic hypertension) + (0.958 if diabetes mellitus) + (1.416 if thrombophilic condition) - (0.487 if multiparae) + (0.093 x BMI). In determining the *a priori* risk in the formula, a value of 2.124 is added to Y' if the patient has had pre-eclampsia previously, and a value of 0 is added to Y' if the patient has not had pre-eclampsia previously. A value of 0.958 is added to Y' if the patient has diabetes mellitus, and a value of 0 is added to Y' if the patient does not have diabetes mellitus. Further, a value of 1.416 is added to Y' if the patient has a thrombophilic condition, and a value of 0 is added to Y' if the patient does not have a thrombophilic condition. In addition, a value of 0.487 is added to Y' if the patient is multiparous, and a value of 0 is added to Y' if the patient is not multiparous.

In one embodiment, a method is provided of determining the need for treating a pregnant female patient comprising: (a) determining the *a priori* risk of developing early pre-eclampsia from maternal factors consisting of (by which is meant the only maternal factors used) previous pre-eclampsia, chronic hypertension, and renal disease; (b) determining the *a posteriori* risk of developing early pre-eclampsia from patient-specific factors consisting of (by which is meant the only patient-specific factors used) mean uterine artery pulsatility index (UtA-PI), mean arterial pressure (MAP), and said *a priori* risk; (c) determining whether said *a posteriori* risk exceeds a pre-determined threshold risk; and (d) determining the need for providing a treatment to said patient if said *a posteriori* risk exceeds said threshold risk, said treatment being selected from: administering a pharmaceutical agent to said patient to prevent, treat, reduce the severity of, or delay the onset of pre-eclampsia or eclampsia; implementing a change in diet or nutrition; inducing labor; performing a Cesarean-section; increasing the frequency of blood pressure measurements for said patient; increasing the frequency of blood testing; and increasing the frequency of fetal monitoring. The patient-specific factors do not comprise placental growth factor (PlGF), vascular endothelial growth factor (VEGF), free β-human chorionic gonadotropin (fβ-hCG), placental protein 13 (PP13), Inhibin A, a disintegrin and metalloprotease-12 (ADAM12), or anti-angiogenic proteins including soluble endoglin (sEng) and the soluble truncated form of the full-length VEGF receptor type-1 (sFltl). The detection rate for detecting early pre-eclampsia is at least about 80%, with a false positive rate of about 10%; or the detection rate for detecting early pre-eclampsia is at least about 90%, with a false positive rate of about 15%.

In one embodiment (not according to the invention), a method is provided of treating a pregnant female patient comprising: (a) determining the *a priori* risk of developing late pre-eclampsia from maternal factors consisting of (by which is meant the only maternal factors used) previous pre-eclampsia, chronic hypertension, diabetes mellitus, thrombophilic condition, multiparity, and body mass index (BMI); (b) determining the *a posteriori* risk of developing late pre-eclampsia from patient-specific factors consisting of (by which is meant the only patient-specific factors used) pregnancy-associated plasma protein-A (PAPP-A) and said *a priori* risk; (c) determining whether said *a posteriori* risk exceeds a pre-determined threshold risk; and (d) providing a treatment to said patient if said *a posteriori* risk exceeds said threshold risk, said treatment being selected from: administering a pharmaceutical agent to said patient to prevent, treat, reduce the severity of, or delay the onset of pre-eclampsia or eclampsia; implementing a change in diet or nutrition; inducing labor; performing a Cesarean-section; or increasing the frequency of blood pressure measurements for said patient; increasing the frequency of blood testing; and increasing the frequency of fetal monitoring. The patient-specific factors do not comprise placental growth factor (PlGF), vascular endothelial growth factor (VEGF), free β-human chorionic gonadotropin (fβ-hCG), placental protein 13 (PP13), Inhibin A, a disintegrin and metalloprotease-12 (ADAM12), or anti-angiogenic proteins including soluble endoglin (sEng) and the soluble truncated form of the full-length VEGF receptor type-1 (sFltl).

In one embodiment, a method is provided of determining the need for a pregnant female patient comprising: (a) determining the *a priori* risk of developing early pre-eclampsia from maternal factors consisting of (by which is meant the only maternal factors used) previous pre-eclampsia, chronic hypertension, and renal disease; (b) determining the *a posteriori* risk of developing early pre-eclampsia from patient-specific factors consisting of (by which is meant the only patient-specific factors used) mean uterine artery pulsatility index (UtA-PI), mean arterial pressure (MAP), and said *a priori* risk; (c) determining whether said *a posteriori* risk exceeds a pre-determined threshold risk; and (d) determining the need for a treatment to said patient if said *a posteriori* risk exceeds said threshold risk, said treatment being selected from: administering a pharmaceutical agent to said patient to prevent, treat, reduce the severity of, or delay the onset of pre-eclampsia or eclampsia; implementing a change in diet or nutrition; inducing labor; performing a Cesarean-section; increasing the frequency of blood pressure measurements for said patient; increasing the frequency of blood testing; and increasing the frequency of fetal monitoring. The patient-specific factors do not comprise placental growth factor (PlGF), vascular endothelial growth factor (VEGF), free β-human chorionic gonadotropin (fβ-hCG), placental protein 13 (PP13), Inhibin A, a disintegrin and metalloprotease-12 (ADAM12), or anti-angiogenic proteins including soluble endoglin (sEng) and the soluble truncated form of the full-length VEGF receptor type-1 (sFltl). The detection rate for detecting early pre-eclampsia is at least about 80%, with a false positive rate of about 10%; or the detection rate for detecting early pre-eclampsia is at least about 90%, with a false positive rate of about 15%. The *a posteriori* risk of developing early pre-eclampsia is determined by calculating: $e^Y/[1 + e^Y]$, where Y is calculated as -0.363 + (2.653 log *a priori* risk) + (12.88 x log MoM mean UtA-PI) + (25.915 x log MoM mean arterial pressure); and where said *a priori* risk is determined by calculating: $e^{Y'}/[1 + e^{Y'}]$, where Y' is calculated as -5.617 + (2.659 if previous pre-eclampsia) + (1.729 if chronic hypertension) + (3.765 if renal disease).

In one embodiment (not according to the invention), a method is provided of treating a pregnant female patient comprising: (a) determining the *a priori* risk of developing late pre-eclampsia from maternal factors consisting of (by which is meant the only maternal factors used) previous pre-eclampsia, chronic hypertension, diabetes mellitus, thrombophilic condition, multiparity, and body mass index (BMI); (b) determining the *a posteriori* risk of developing late pre-eclampsia from patient-specific factors consisting of (by which is meant the only patient-specific factors used) pregnancy-associated plasma protein-A (PAPP-A) and said *a priori* risk; (c) determining whether said *a posteriori* risk exceeds a pre-determined threshold risk; and (d) providing a treatment to said patient if said *a posteriori* risk exceeds said threshold risk, said treatment being selected from: administering a pharmaceutical agent to said patient to prevent, treat, reduce the severity of, or delay the onset of pre-eclampsia or eclampsia; implementing a change in diet or nutrition; inducing labor; performing a Cesarean-section; or increasing the frequency of blood pressure measurements for said patient; increasing the frequency of blood testing; and increasing the frequency of fetal monitoring. The patient-specific factors do not comprise placental growth factor (PIGF), vascular endothelial growth factor (VEGF), free β-human chorionic gonadotropin (fβ-hCG), placental protein 13 (PP13), Inhibin A, a disintegrin and metalloprotease-12 (ADAM12), or anti-angiogenic proteins including soluble endoglin (sEng) and the soluble truncated form of the full-length VEGF receptor type-1 (sFltl). The *a posteriori* risk of developing late pre-eclampsia is determined by calculating: $e^Y/[1 + e^Y]$, where Y is calculated as 0.328 + (2.205 x log *a priori* risk) - (1.307 x log MoM PAPP-A); and where said *a priori* risk is determined by calculating: $e^{Y'}/[1 + e^{Y'}]$, where Y' is calculated as -6.135 + (2.124 if previous pre-eclampsia) + (1.571 if chronic hypertension) + (0.958 if diabetes mellitus) + (1.416 if thrombophilic condition) - (0.487 if multiparae) + (0.093 x BMI).

## EXAMPLES

The following examples describe specific aspects of the invention to illustrate the invention but should not be construed as limiting the invention, as the examples merely provide specific methodology useful in the understanding and practice of the invention and its various aspects.

## EXAMPLE 1

The following study was performed in order to develop a logistic regression-based predictive model for early and late onset pre-eclampsia.

## Methods and patients

A prospective cohort composed of singleton pregnancies underwent routine first-trimester screening at the Department of Maternal-Fetal Medicine at Hospital Clinic Barcelona. The local Ethics Committee approved the study protocol and each patient provided written informed consent. Gestational age in all pregnancies was calculated based on the crown-rump length (CRL) at first-trimester ultrasound. Maternal characteristics and medical history were recorded and blood pressure, UtA, and plasma concentrations of PAPP-A, and fβ-HCG were measured in the first trimester.

Between May 2007 and October 2009, a total of 5,759 women underwent examination. Of these participants, a total of 589 (10.2%) were excluded for the following reasons (non-exclusively): missing outcome data (n=525), major fetal defects or chromosomopathies (n=25), miscarriage or fetal death before 24 weeks (n=80), and termination of pregnancy in the absence of medical indication (n=21). After these participants were excluded, 5,170 cases remained for the study.

Maternal characteristics and medical history were prospectively recorded at the time of first trimester ultrasound (11.0-13.6 weeks) via a patient questionnaire. Characteristics recorded were: medical and obstetric history, maternal age, ethnicity, smoking status, parity, height, and weight.

A nurse measured blood pressure automatically with a calibrated OMRON M6 Comfort (OMRON Corporation, Kyoto, Japan) device in our outpatient clinics according to standard procedure. Blood pressure was measured in one arm (right or left) without distinction while women were seated, and after a 5-minute rest. Mean arterial pressure (MAP) was calculated as: diastolic blood pressure + (systolic - diastolic)/3.

UtA evaluation was performed transvaginally during the first trimester ultrasound. Both uterine artery pulsatility indices (PIs) were automatically measured and mean uterine artery PI (UtA-PI) was calculated.

Maternal serum PAPP-A and free β-HCG were measured using the AUTODELFIA XPRESS analyzer (Perkin-Elmer Life Sciences, Turku, Finland) between 8 and 12 weeks of gestation. Thereafter, these levels were converted to multiples of the expected normal median (MoM), which were corrected for CRL, maternal age, body-mass index (BMI), smoking and diabetes status, and ethnicity.

Pre-eclampsia was defined as systolic BP ≥140 mmHg and/or diastolic BP ≥90 mmHg on at least two occasions 4 hours apart, developing after 20 weeks of gestation in previously normotensive women, and proteinuria >300 mg in a 24-hour urine specimen. Early pre-eclampsia was defined as pre-eclampsia requiring delivery before 34 weeks. Doctors that made the diagnosis were blinded to the study parameters obtained during the first trimester.

Statistical analysis

The Mann-Whitney U-test and Pearson $\chi 2$-squared test were performed to make univariate comparisons of quantitative and qualitative variables, respectively, between groups. A post-hoc Bonferroni correction was conducted to maintain a type-I error of 0.05 (p-value = 0.025).

Logarithmic transformations were performed to normalize mean UtA-PI and MAP. Expected log values were calculated for all cases using a linear regression analysis of unaffected cases and included the following co-variables: maternal age at first-trimester ultrasound (years), crown-rump length at first trimester ultrasound (millimeters), maternal height (centimeters) and weight at examination (kilograms), parity (nulliparous vs. multiparous), smoking status upon examination (0, 1-9, 10-19, and 20+ cigarettes/day), and ethnicity (white-European, South-America, Black, and Other). Each individual observed value was expressed as a MoM of the expected value.

Logistic regression was used to estimate each woman's *a priori* risk with respect to the following covariates: previous medical history of diabetes; chronic hypertension; renal or autoimmune diseases; congenital and acquired thrombophilic conditions; previous obstetric history of preeclampsia or intrauterine growth restriction; maternal age; BMI $(kg/m^2)$; smoking (cigarettes/days); ethnicity and parity.

Logistic regression analysis was performed to estimate the individual risks for early and late preeclampsia with respect to the following co-variables: *a priori* risk (log-transformed), log MoM mean UtA-PI, log MoM MAP, log MoM PAPP-A and log MoM fβHCG. [0062] Receiver operator characteristic (ROC) curves were performed to analyze model performance, which was expressed as detection rates for different cut-offs of false positive rates.

In all regression models, stepwise forward algorithms were performed to select variables at a p-value cut-off of 0.05. Goodness-of-fit models were assessed by calculating Nagelkerke's $R^2$.

The statistical package IBM SPSS 18.0 (New York, USA) was used to conduct all the statistical analyses and graphs were generated with MedCalc software (Mariakerke, Belgium).

Results

Among the 5,170 women included in the study, 136 (2.6%) developed preeclampsia, including 110 (2.1%) cases of late preeclampsia and 26 (0.5%) cases of early preeclampsia. Table 1 shows the epidemiological and clinical characteristics of the population by study group.

Table 1

| | Unaffected (n=5034) | Late PE (n=110) | Early PE (n=26) |
|---|---|---|---|
| **Age in years; median (IQR)** | 32 (28-35.4) | 33.2 (29-36.3) | 31.3 (29.9-36.5) |
| **BMI in Kg/m$^2$; median (IQR)** | 24 (22.7-24.7) | 24.6 (23.5-26.4)$^\#$ | 24.4 (22.7-28) |
| **Ethnicity** | | | |
| White-European; n (%) | 3757 (74.6) | 73 (66.4) | 15 (57.7) |
| Black; n (%) | 22 (0.4) | 1 (0.9) | 1 (3.8) |
| South American; n (%) | 784 (15.6) | 28 (25.5) | 6 (23.1) |
| Others; n (%) | 471 (9.4) | 8 (7.3) | 4 (15.4) |
| **Smoking status** | | | |
| No; n (%) | 4637 (92.1) | 100 (90.9) | 24 (92.3) |
| <10 cigarettes/day; n (%) | 107 (2.1) | 4 (3.6) | 0(0) |
| 10-20 cigarettes/day; n (%) | 245 (4.9) | 4 (3.6) | 1 (3.8) |
| >20 cigarettes/day; n (%) | 45 (0.9) | 2 (1.8) | 1 (3.8) |

(continued)

| Medical history | | | |
|---|---|---|---|
| Chronic hypertension; n (%) | 48 (1) | 10 (9.1)# | 4 (15.4)+ |
| Diabetes mellitus; n (%) | 88 (1.7) | 7 (6.4)# | 0(0) |
| Renal disease; n (%) | 6 (0.1) | 0 | 3 (11.5)+* |
| Autoimmune disease; n (%) | 68 (1.4) | 4 (3.6) | 1 (3.8) |
| Coagulation disorders; n (%) | 40 (0.8) | 4 (3.6)# | 0(0) |
| **Obstetric history** | | | |
| Nulliparous; n (%) | 2971 (59) | 70 (63.6) | 14 (53.8) |
| Previous preeclampsia; n (%) | 28 (0.6) | 10 (9.1)# | 5 (19.2)+ |
| IUGR; n (%) | 28 (0.6) | 1 (0.9) | 3 (11.5)+* |
| **Mean BP; median (IQR)** | 78.5 (74.1-83.1) | 79.4 (74.9-84.1) | 85.7 (80-89.7)+* |
| **Mean UtA PI; median (IQR)** | 1.67 (0.53-1.25) | 1.68 (1.54-1.84) | 2.23 (1.75-3)+* |
| **Maternal serum biochemistry** | | | |
| PAPP-A (MoM); median (IQR) | 1.06 (0.53-1.25) | 0.55 (0.28-1.05)# | 0.87 (0.44-1.24) |
| β-HCG (MoM); median (IQR) | 1 (0.63-1.16) | 0.96 (0.55-1.15) | 0.92 (0.5-1.04) |

IQR: Interquartile range; BMI: Body-mass index; PE: Preeclampsia; IUGR: intrauterine growth restriction; PAPP-A: Pregnancy associated plasma protein-A; β-HCG: β-human chorionic gonadotropin; BP, blood pressure; UtA PI, uterine artery pulsatility indices; MoM: Multiple of the expected normal median. Significant comparisons between unaffected and late PE (#); unaffected and early PE (+); and late and early PE (*)

The following formula best fit the expected Log mean UtA PI: 0.668018 - (0.002772*CRL) - (0.001536*height) - (0.001151*maternal age); $R^2 = 4.6\%$.

The following formula best fit the expected Log MAP: 1.803485 + (0.002990*BMI) + (0.000645*maternal age) - (0.00421 if South-American); $R^2 = 13.1\%$.

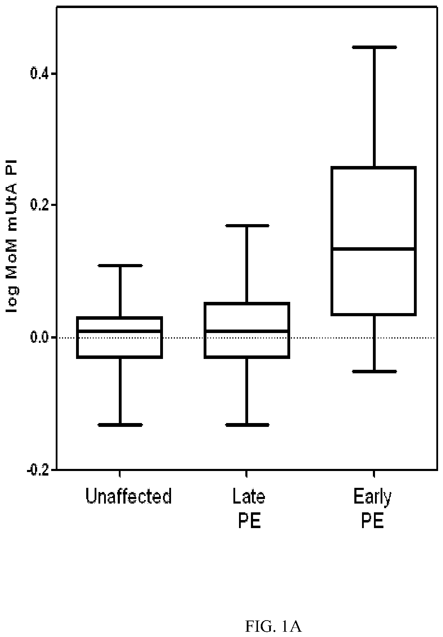

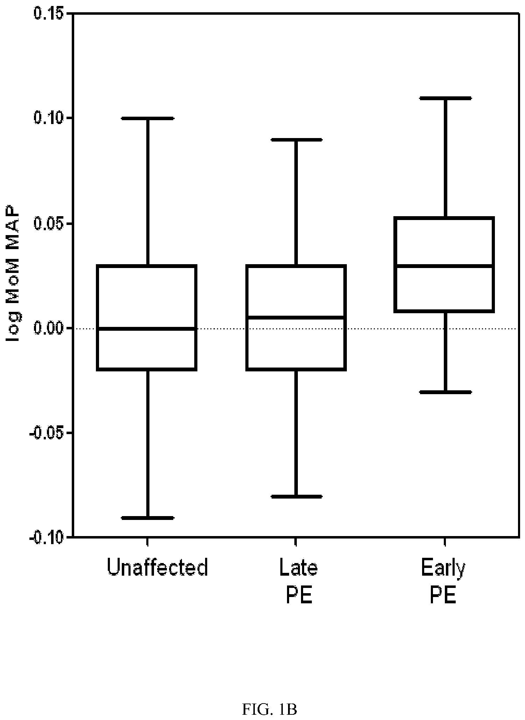

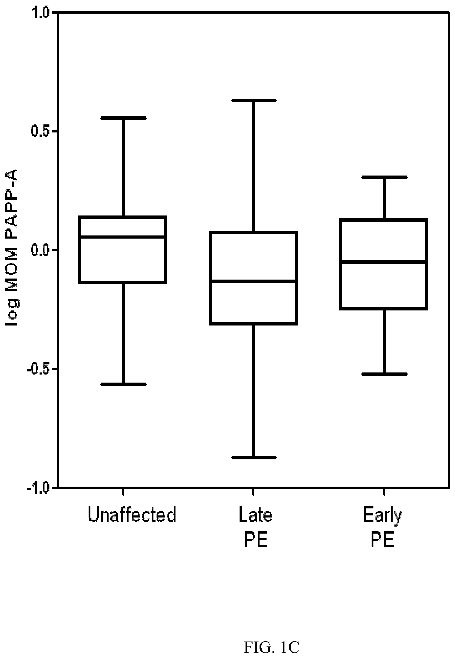

Figures 1a-1c show the distribution in log MoM of mean UtA PI, MAP, and PAPP-A among the different study groups. The log MoM mean UtA-PI was significantly greater in the early PE group compared to unaffected (p<0.001) and late PE groups (p=0.001). Similarly, log MoM MAP was significantly higher in the early PE group than in the unaffected (p=0.001) and late PE (p<0.001) groups. Log MoM PAPP-A was significantly lower in the late PE group compared to the unaffected group (p <0.001).

The following model best fit the *a priori* risk for late and early PE [*a priori* risk=$e^y/(1+e^y)$]:

Late PE Y = -6.135 + (2.124 if previous PE) + (1.571 if chronic hypertension) + (0.958 if diabetes mellitus) + (1.416 if thrombophilic condition) - (0.487 if multiparae) + (0.093*BMI); $R^2 = 7.9\%$.

Early PE Y = 5.617 + (2.659 if previous PE) + (1.729 if chronic hypertension) + (3.765 if renal disease); $R^2 = 13.4\%$.

The following models best fit the patient-specific *a posteriori* risk (*a posteriori* risk = $e^y/[1+e^y]$):

Late PE Y = 0.328 + (2.205 * log *a priori* risk) - (1.307 * log MoM PAPP-A); $R^2 = 10.1\%$

Early PE Y = -0.363 + (2.653 * log *a priori* risk) + (12.88 * log MoM mean UtA-PI) + (25.915 * log MoM MAP); $R^2 = 36.6\%$.

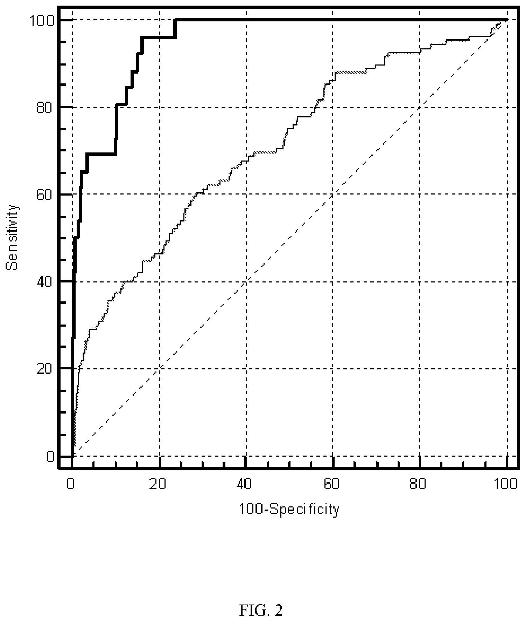

Performance of the model was analyzed using an ROC curve, with performance expressed as detection rates for different cut-offs of false positive rates. Figure 2 shows the ROC for late PE (AUC: 0.710; 95% CI: 0.658-0.763) and early PE (AUC: 0.951; 95% CI: 0.925-0.977) models.

The diagnostic performance for late and early PE for false positive rates (FPRs) of 5%, 10%, and 15% is presented in Table 2 below.

Table 2

| | Risk cut-off | Prevalence of + (%) | DR | FPR | +LHR | -LHR |
|---|---|---|---|---|---|---|
| Late PE | >1/14 | 6.3 | 29.4 | | 5.39 | 0.75 |
| | >1/18 | 11.4 | 39,6 | 10 | 3.61 | 0.7 |
| | >1/22 | 16.4 | 42.2 | 15 | 2.73 | 0.68 |
| Early PE | >1/88 | 5.8 | 69.2 | | 12.7 | 0.33 |
| | >1/180 | 10.8 | 80.8 | 10 | 7.74 | 0.21 |
| | >1/308 | 15.8 | 92.3 | 15 | 5.98 | 0.09 |
| PE: preeclampsia; DR: Detection rate; FPR: False positive rate; +LHR: Positive likelihood ratio; -LHR: Negative likelihood ratio | | | | | | |

The results of this study demonstrate that the combination of specific maternal and patient-specific factors can be utilized in order to predict both early and late onset pre-eclampsia in women in the first trimester of pregnancy.

**EXAMPLE 2**

The application of a specific patient's data to the models described in Example 1 is provided. The models are applied to a 35-year-old woman with a prothrombin gene mutation, but no other medical conditions, who underwent her first pregnancy. At the time of the first-trimester ultrasound (CRL: 65 mm), the patient's height was 165 cm, and her weight was 65 kg (BMI 23.8 kg/m$^2$). The patient had a mean UtA-PI of 1.85, a MAP of 90 mmHg, and a PAPPA-P of 0.87 MoM. The following results are based on the data obtained from this patient.

The expected log mean UtA-PI is: 0.668018 - (0.002772 * 65) - (0.001536 * 165) - (0.001151 * 35) = 0.194

The log MoM mean UtA PI is: log(1.85) - 0.194 = 0.073

The expected log MAP is: 1.803485 + (0.00299 * 23.8) + (0.000645 * 35) = 1.897

The log MoM MAP is: log(90) - 1.897 = 0.057

The *a priori* odds for early PE is: Y = -5.617

The *a priori* risk = $e^{-5.617}/(1+e^{-5.617})$ = 0.0036

The *a posteriori* odds for early PE is: Y = -0.363 + (2.653 * log(0.0036)+(12.88 * 0.073) + (25.915 * 0.057) = -4.4287

The *a posteriori* risk = $e^{-4.4287}/(1 + e^{-4.4287})$ = 0.0118 = 1/85.

A patient having all of the same results, but with a mean UtA-PI of 2.5 would have a risk of 1/17 for early PE.

**Claims**

1. A method of screening for and predicting the likelihood that a pregnant female patient will develop early pre-eclampsia and determining the need for providing a treatment to said patient comprising:

(a) determining the *a priori* risk of developing early pre-eclampsia from maternal factors comprising previous pre-eclampsia, chronic hypertension, and renal disease;
(b) determining the *a posteriori* risk of developing early pre-eclampsia from patient-specific factors comprising mean uterine artery pulsatility index (UtA-PI), mean arterial pressure (MAP), and said *a priori* risk;
(c) determining whether said *a posteriori* risk exceeds a pre-determined threshold risk; and
(d) determining the need for providing a treatment to said patient if said *a posteriori* risk exceeds said threshold risk, said treatment being selected from: administering a pharmaceutical agent to said patient to prevent, treat, reduce the severity of, or delay the onset of pre-eclampsia or eclampsia; implementing a change in diet or nutrition; inducing labor; performing a Cesarean-section; increasing the frequency of blood pressure measurements for said patient; increasing the frequency of blood testing; and increasing the frequency of fetal monitoring,

wherein said *a posteriori* risk is determined by $e^Y/[1 + e^Y]$, where

$$Y = -0.363 + (2.653 \log a\ priori\ \text{risk}) + (12.88 \times \log \text{MoM mean UtA-PI}) + (25.915 \times \log \text{MoM mean arterial pressure});$$

where said *a priori* risk is determined by $e^{Y'}/[1 + e^{Y'}]$, where

$$Y' = -5.617 + (2.659 \text{ if previous pre-eclampsia}) + (1.729 \text{ if chronic hypertension}) + (3.765 \text{ if renal disease}).$$

2. The method according to claim 1, wherein said maternal factors consist of previous pre-eclampsia, chronic hypertension, and renal disease.

3. The method according to claim 1, wherein said patient-specific factors consist of mean uterine artery pulsatility index (UtA-PI), mean arterial pressure, and said *a priori* risk.

4. The method according to claim 1, wherein said patient-specific factors do not comprise placental growth factor (PIGF), vascular endothelial growth factor (VEGF), free β-human chorionic gonadotropin (fβ-hCG), placental protein 13 (PP13), Inhibin A, a disintegrin and metalloprotease-12 (ADAM12), or anti-angiogenic proteins including soluble endoglin (sEng) and the soluble truncated form of the full-length VEGF receptor type-1 (sFltl).

5. The method according to claim 1, wherein said patient-specific factors do not comprise biochemical markers.

6. The method according to claim 1, wherein said *a priori* risk is calculated utilizing coefficients of binary variables for each of said maternal factors, said coefficients being generated utilizing logistic regression analysis.

7. The method according to claim 1, wherein said *a posteriori* risk is calculated utilizing coefficients for each of said patient-specific factors, said coefficients being generated utilizing logistic regression analysis.

8. The method according to claim 1, wherein said determining step (c) has a detection rate of at least about 80% and a false positive rate of about 10%, preferably wherein said determining step (c) has a detection rate of at least about 90% and a false positive rate of about 15%.

**Patentansprüche**

1. Verfahren zum Screening auf und Vorhersagen der Wahrscheinlichkeit, dass eine schwangere Patientin Präeklampsie entwickelt und Bestimmen des Bedarfs zur Behandlung der Patientin, umfassend:

(a) Bestimmen des *A-priori*-Risikos der Entwicklung einer frühen Präeklampsie aus mütterlichen Faktoren, umfassend vorhergehende Präeklampsie, chronische Hypertonie und Nierenerkrankung;
(b) Bestimmen des *A-posteriori*-Risikos der Entwicklung einer frühen Präeklampsie aus patientenspezifischen Faktoren, die den mittleren Uterusarterien-Pulsatilitätsindex (UtA-PI), den mittleren arteriellen Druck (MAP) und das A-*priori-Risiko* umfassen;
(c) Bestimmen, ob das *A-posteriori-Risiko* ein vorbestimmtes Schwellenrisiko übersteigt; und
(d) Bestimmen des Bedarfs an der Behandlung der Patientin, wenn das *A-posteriori*-Risiko das Schwellenrisiko übersteigt, wobei die Behandlung ausgewählt ist aus: Verabreichen eines pharmazeutischen Mittels an die Patientin zur Vorbeugung, Behandlung, Verringerung der Schwere oder Verzögerung des Auftretens von Präeklampsie oder Eklampsie; Umsetzung einer Änderung von Kost oder Ernährung; Einleiten der Wehen; Durchführen eines Kaiserschnitts; Erhöhen der Häufigkeit von Blutdruckmessungen für die Patientin; Erhöhung der Häufigkeit von Bluttests; und Erhöhung der Häufigkeit der Fötusüberwachung,

wobei das *A-posteriori*-Risiko durch e$^Y$/[1 + e$^Y$] bestimmt wird, wobei

```
Y = -0,363 + (2,653 log A-priori-Risiko) + (12,88
x log MoM-Mittelwert von UtA-PI) + (25,915 x log MoM
mittlerer arterieller Druck);
```

wobei das A-priori-Risiko durch e$^{Y'}$/[1 + e$^{Y'}$] bestimmt wird, wobei

```
Y'= -5,617 + (2,659 bei vorhergehender
Präeklampsie) + (1,729 bei chronischer Hypertonie) +
(3,765 bei Nierenerkrankung).
```

2. Verfahren nach Anspruch 1, wobei die mütterlichen Faktoren aus vorhergehender Präeklampsie, chronischer Hypertonie und Nierenerkrankung bestehen.

3. Verfahren nach Anspruch 1, wobei die patientenspezifischen Faktoren aus dem mittleren Uterusarterien-Pulsatilitätsindex (UtA-PI), dem mittleren arteriellen Druck und dem A-priori-Risiko bestehen.

4. Verfahren nach Anspruch 1, wobei die patientenspezifischen Faktoren kein(en) plazentalen Wachstumsfaktor (PlGF), vaskulären endothelialen Wachstumsfaktor (VEGF), freies β-humanes Choriongonadotropin (fβ-hCG), Plazentaprotein 13 (PP13), Inhibin A, Disintegrin und Metalloprotease-12 (ADAM 12) oder anti-angiogene Proteine einschließlich löslichem Endoglin (sEng) und die lösliche verkürzte Form des VEGF-Rezeptors Typ-1 voller Länge (sFlt1) umfassen.

5. Verfahren nach Anspruch 1, wobei die patientenspezifischen Faktoren keine biochemischen Marker umfassen.

6. Verfahren nach Anspruch 1, wobei das *A-priori*-Risiko unter Verwendung von Koeffizienten von binären Variablen für jeden der mütterlichen Faktoren berechnet wird, wobei die Koeffizienten unter Verwendung einer logistischen Regressionsanalyse erzeugt werden.

7. Verfahren nach Anspruch 1, wobei das *A-posteriori*-Risiko unter Verwendung von Koeffizienten für jeden der patientenspezifischen Faktoren berechnet wird, wobei die Koeffizienten unter Verwendung einer logistischen Regressionsanalyse erzeugt werden.

8. Verfahren nach Anspruch 1, wobei der Bestimmungsschritt (c) eine Nachweisrate von mindestens etwa 80% und eine falsch positive Rate von etwa 10% aufweist, wobei der Bestimmungsschritt (c) vorzugsweise eine Nachweisrate von mindestens etwa 90% und eine falsch positive Rate von etwa 15% aufweist.

**Revendications**

1. Procédé de dépistage et de prédiction de la probabilité qu'une patiente enceinte développera une pré-éclampsie précoce et de détermination de la nécessité de fournir un traitement à cette patiente, comprenant les étapes consistants à :

(a) déterminer le risque *a priori* de développer une pré-éclampsie précoce à partir de facteurs maternels comprenant une pré-éclampsie antérieure, une hypertension chronique et une maladie rénale ;
(b) déterminer le risque *a posteriori* de développer une pré-éclampsie précoce à partir de facteurs spécifiques à la patiente comprenant l'indice de pulsatilité de l'artère utérine moyen (UtA-PI), la pression artérielle moyenne (MAP) et ledit risque *a priori* ;
(c) déterminer si ledit risque *a posteriori* dépasse un seuil de risque prédéterminé ; et
(d) déterminer la nécessité

de fournir un traitement à ladite patiente si ledit risque *a posteriori* dépasse ledit seuil de risque, ledit traitement étant choisi parmi : administrer un agent pharmaceutique à ladite patiente pour prévenir, traiter, réduire la gravité de, ou retarder l'apparition de la pré-éclampsie ou de l'éclampsie ; mettre en oeuvre un changement de régime alimentaire ou de nutrition ; inciter au travail ; effectuer une césarienne ; augmenter la fréquence des mesures de la pression sanguine pour ladite patiente; augmenter la fréquence des tests sanguins; et augmenter la fréquence de surveillance foetale,

dans lequel ledit risque *a posteriori* est déterminé par $e^Y/[1 + e^Y]$, où

$$Y = -0,363 + (2,653 \log \text{ de risque } a\ priori) + (12,88 \times \log \text{ MoM moyenne UtA-PI}) + (25,915 \times \log \text{ Mom Pression artérielle moyenne}) ;$$

où ledit risque *a priori* est déterminé par $e^{Y'}/[1 + e^{Y'}]$,
où

$$Y'= -5,617 + (2,659 \text{ si pré-éclampsie antérieure}) + (1,729 \text{ si hypertension chronique}) + (3,765 \text{ si maladie rénale}).$$

2. Procédé selon la revendication 1, dans lequel lesdits facteurs maternels consistent en une pré-éclampsie antérieure, une hypertension chronique et une maladie rénale.

3. Procédé selon la revendication 1, dans lequel lesdits facteurs spécifiques à la patiente consistent en un indice de pulsatilité de l'artère utérine moyen (UtA-PI), une pression artérielle moyenne et ledit risque *a priori.*

4. Procédé selon la revendication 1, dans lequel lesdits facteurs spécifiques à la patiente ne comprennent pas le facteur de croissance placentaire (PIGF), le facteur de croissance endothélial vasculaire (VEGF), la gonadotrophine chorionique β-humaine libre (fβ-hCG), la protéine placentaire 13 (PP13), l'Inhibin A, la désintégrine et la métalloprotéase-12 (ADAM12), ou des protéines anti-angiogéniques comprenant une endogline soluble (sEng) et la forme tronquée soluble du récepteur VEGF complet de type 1 (sFlt1).

5. Procédé selon la revendication 1, dans lequel lesdits facteurs spécifiques à la patiente ne comprennent pas de marqueurs biochimiques.

6. Procédé selon la revendication 1, dans lequel ledit risque *a priori* est calculé en utilisant des coefficients de variables binaires pour chacun desdits facteurs maternels, lesdits coefficients étant générés en utilisant une analyse de régression logistique.

7. Procédé selon la revendication 1, dans lequel ledit risque *a posteriori* est calculé en utilisant des coefficients pour chacun desdits facteurs spécifiques à la patiente, lesdits coefficients étant générés en utilisant une analyse de régression logistique.

8. Procédé selon la revendication 1, dans lequel ladite étape de détermination (c) a un taux de détection d'au moins environ 80% et un taux de faux positifs d'environ 10%, de préférence dans lequel ladite étape de détermination (c) a un taux de détection d'au moins environ 90% et un taux de faux positifs d'environ 15%.

FIG. 1A

FIG. 1B

FIG. 1C

FIG. 2